# EUROPEAN PATENT SPECIFICATION

(11) **EP 1 807 020 B1**
(45) Date of publication and mention of the grant of the patent: **27.04.2011**
(21) Application number: 05779734.2
(22) Date of filing: 08.08.2005
(51) Int. Cl.: A61F 2/06

(54) **MEDICAL DEVICES COATED WITH DIAMOND-LIKE CARBON**
MIT DIAMANTARTIGEM KOHLENSTOFF BESCHICHTETE MEDIZINPRODUKTE
DISPOSITIFS MEDICAUX A REVETEMENT A BASE DE CARBONE SOUS FORME DE DIAMANT AMORPHE

(30) Priority: 08.10.2004 US 961666
(43) Date of publication of application: 18.07.2007
(73) Proprietor: Boston Scientific Limited, Barbados, West Indies (BB)
(72) Inventor: BURGMEIER, Robert, Plymouth, Minnesota 55447 (US); HORN, Daniel, Shoreview, Minnesota 55126 (US)
(74) Representative: Schildberg, Peter
(86) International application number: PCT/US2005/028284
(87) International publication number: WO 2006/041558

(56) References cited:
- EP-A- 0 876 821
- US-A1- 2004 148 007
- PATENT ABSTRACTS OF JAPAN vol. 1999, no. 09, 30 July 1999 (1999-07-30) -& JP 11 106920 A (NISSIN ELECTRIC CO LTD), 20 April 1999 (1999-04-20)
- PATENT ABSTRACTS OF JAPAN vol. 2003, no. 12, 5 December 2003 (2003-12-05) & JP 2003 310744 A (KEIO GIJUKU), 5 November 2003 (2003-11-05)
- PATENT ABSTRACTS OF JAPAN vol. 2003, no. 05, 12 May 2003 (2003-05-12) & JP 2003 000527 A (PENTAX CORP), 7 January 2003 (2003-01-07)

## Description

### FIELD OF THE INVENTION

The invention pertains to medical devices, especially catheters and the like having sliding surfaces that are coated with diamond-like carbon (DLC).

### BACKGROUND OF THE INVENTION

A number of medical devices have surfaces that, in normal use, are subjected to sliding actions. Sliding actions include deployment of catheters, endoscopes and the like through body lumens, over wires, or through lumens of other devices. Sliding resistance may be due to inherent properties of the substrate material, to specific properties of the substrates at their interface, such as surface roughness, or to interaction of the surfaces with other materials, especially liquids such as blood, water, saline, lubricants or the like.

Coating portions of devices such as balloon catheters with certain hydrophilic or hydrophobic lubricious materials to reduce sliding resistance is well known. However, this has not been entirely satisfactory since such materials often have poor durability during use, or must be provided in a manner in which a significant fraction of the total wall thickness of the device must be devoted to lubrication property, at the sacrifice of optimal performance properties such as wall strength or plasticity.

Diamond-like carbon (DLC) is a form of carbon deposited from carbon plasmas. Devices and processes for depositing DLC carbon coatings onto various substrates are known.

US 5,858,477, Veersamy et al, describes DLC coatings applied on magnetic recording material films.

Substrates which include a hydrophobic coating system including both DLC and fluoro-alkyl silane layers are described in US 6,531,182, Veersamy et al.

US 6,562,445, Iwamura, describes wear resistant multilayer coating film comprising a layer of DLC over a low hardness carbon-layer. On substrates of this document the underlying substrate may be metal alloys, ceramics (including glass), silicon and resin materials.

US 6,696,157, David et al, describe diamond-like glass films which incorporate silicon and oxygen as well as carbon. Substrates used in the examples include silicon wafers, quartz slides, acrylate coated optical fibers, polyethylene heat shrink film, poly(methyl methacrylate) channeled plates and capillaries and poly (bicyclopentadiene) capillaries.

US 6,660,340, Kirkpatrick, describes a method and apparatus for enhancing adhesion of DLC to a substrate by pre-processing the substrate in a carbon ion beam.

A variety of devices and techniques for exposing substrates to plasma environments are described in US 5,705,233, US 5,604,038, US 5,908,539, US 6,054,018, US 6,082,292 and US 6,096,564, all assigned to Wisconsin Alumni Research Foundation.

In the area of medical devices, coatings for bearing and articulation surfaces of prosthetic joints are described in US 5,593,719, Dearnaley et al, and US 6,709,463, Pope et al. The coatings may be diamond-like carbon.

Implantable medical devices having a coating on an inner or outer layer and a sensor incorporated into or under the coating are described in US 6,592,519, Martinez. The implantable device may be a drug delivery device which includes a coated drug delivery catheter of some uncertain structure and material. The coating may be diamond or a diamond-like material.

US 6,607,598, Schwartz et al, describes devices useful for protecting a medical device during a coating process. The coatings which may be applied to the medical device may include ionization deposited materials such as DLC.

Stents coated with DLC are described in US 6,572,651 B1, DeScheerder et al.

It has not previously been proposed to modify sliding surfaces of medical devices with DLC, or to reduce sliding resistance by such a modification. It has not previously been proposed to form medical catheters, balloons, stent placement structures or guide wires with a coating of DLC.

US 2004/148007 A1 discloses a polymeric catheter having a sheath. A coating which may comprise DLC is attached to the inner circumference of said sheath covering the stent.

### SUMMARY OF THE INVENTION

The invention pertains to a medical device comprising a catheter having a tubular polymeric portion comprising a guide wire lumen, having an inner lumen wall, the tubular portion being only of polymeric material. The inner lumen wall is coated with a diamond hard coating (DLC). The tubular polymeric portion further has an exterior surface and the exterior surface has a coating thereon obtained by exposing the surface to a plasma, and wherein said plasma comprised a fluorinating or oxidizing gas.

The invention pertains further to a method of treating a surface of a polymeric medical device comprising a catheter substrate comprising:
- enclosing the polymeric medical device substrate in a reaction chamber with the surface to be coated exposed to the environment of the reaction chamber;
- evacuating the reaction chamber to a base level;
- supplying a hydrocarbon DLC depositing gas to and establishing a selected hydrocarbon DLC depositing hydrocarbon gas pressure in the reaction chamber; and
- igniting a cold plasma in the gas in the chamber and exposing the substrate surface to the plasma for a selected period of time sufficient to form a layer of DLC thereon.

The medical device substrate is a tubular substrate formed only of polymeric material and having an inner lumen, the hydrocarbon DLC depositing gas is fed at least through said lumen, the plasma is ignited in the gas within said lumen, and the medical device substrate has an outer surface and a different film forming plasma generating gas comprising oxygen and fluorine is fed around the outer surface.

### BRIEF DESCRIPTION OF THE DRAWINGS

Fig. 1 is a schematic depiction of a plasma treatment apparatus that may be employed in the invention.
Fig. 2 schematically illustrates an plasma reaction chamber that may be employed for continuous or semi-continuous cold plasma treatment of wire, tubing, and the like.
Fig. 3 illustrates a reaction chamber design that allows for rotation of the substrate and/or for plasma treatment of an internal lumen surface.
Fig. 4 illustrates an alternative arrangement of electrodes for a plasma reaction chamber employed in the invention.
FIG. 5 is a longitudinal cross-sectional view of a balloon catheter having several sliding surfaces that may be modified to incorporate a DLC coating in accordance with the invention.

### DETAILED DESCRIPTION

All published documents, including all US patent documents, mentioned anywhere in this patent are hereby expressly incorporated herein by reference in their entirety. Any copending patent applications, mentioned anywhere in this application are also hereby expressly incorporated herein by reference in their entirety.

The present invention relates to the surface modification of medical devices which are at least in part formed from a polymeric composition by utilizing a gaseous DLC-depositing plasma to modify the surface of the polymeric composition.

The surface modification process finds utility for a variety of medical devices including, but not limited to, vascular catheters including guide catheters and catheters for angioplasty, and other devices for use in urological procedures, for use in the biliary duct, for neurological procedures, for use in the reproductive system, for delivery of medical devices such as stents, etc.

Using the surface modification process according to the invention, the surface of medical devices may be modified to lower sliding resistance, as well as to increase the durability of the device surface, while the bulk properties of the substrate remain substantially unchanged. The coating may be on inside surfaces or on outside surfaces of the substrate. In particular embodiments the surface being coated is polymeric. In others it maybe metal or ceramic.

Applications of the invention are seen for any tubular or wire-like surface where a durable low sliding friction surface is needed, especially where the thickness dimension of the device must be minimal. The coating may be on the outer surface, or an inner tubular surface, and it may be continuous or discontinuous.

A sliding surface of any medical device may be modified using the techniques described herein. The present invention finds particular utility for catheter assemblies. Catheter assemblies are employed in a wide range of procedures and are used for example, for procedures in vasculature (including coronary vasculature), in the biliary duct, in the neurological system, in the urinary tract, in the reproductive system, etc. as well as guide catheters and delivery systems for medical devices such as stent delivery systems. By way of non-limiting example, the present invention may be employed to modify catheter shaft inner or outer surfaces, as well as such surfaces of balloons. Stent sleeves or other stent protecting structures may also be advantageously provided with DLC coatings. Guide wires may also be advantageously coated with DLC to reduce sliding friction in the body and to reduce lumen friction when the catheter is passed over the wire.

The DLC coating can also be advantageously employed on surfaces that must be peeled from contact with another. Some stent protection structures work in such a way. A DLC coating can reduce adhesion at the interface of such surfaces.

Especially with polymer materials it is preferred that a cold plasma technique is employed that will not cause significant damage due to heating. In a cold plasma treatment, the temperature of the plasma is relatively low, e.g. from about 10 to about 120°C, suitably 20-60°C. The treatment may be carried out in a vacuum, with the substrate surface to be modified being placed within the vacuum, suitably inside an evacuated chamber. The strength of the vacuum is not limited, provided that it is sufficient to allow plasma coating to occur. In some embodiments the vacuum is at a pressure of 0.005 Torr or less. However it may also be possible to generate DLC depositing or reactive plasma at or near atmospheric pressure and temperature, leaving open the possibility for a continuous coating operation, for instance, in an extrusion line downstream of the extruder and coolant tank, but before the material has been collected at a takeup or cutting station.

In an exemplary process a DLC modification gas is introduced at a controlled rate into a vacuum chamber in which the surface to be modified is situated, or through which the substrate surface is passed. The DLC modification gas is any gas, or mixture of gases, that forms a carbon depositing plasma or reacts with the substrate surface to leave a diamond-like coating thereon. A radio frequency signal is applied via an external antenna to form the plasma. It will be clear to the skilled person that the appropriate frequency may be selected, depending upon the particular DLC modification gas employed. Generally frequencies of the order of 10 kHz to 10 MHz are useful in the present invention, although lower or higher frequencies may be employed, depending upon the substance being employed to modify the surface. The power of the RF signal is not limited, provided that it is sufficient to ignite the plasma and promote coating. A power of from 50 to 100 W may be employed. The plasma is ignited within the chamber and maintained for a selected time at a pre-selected power setting. Once the treatment is complete, the radio frequency is switched off to extinguish the plasma. The chamber is then be flushed, and the products retrieved. As a result of the procedure, a thin layer of DLC is attached to the surface to be modified. The layer thickness may be from about 10 to 10,000 Å, for instance from 50 to 5000 Å.

In some embodiments of the present invention, the DLC surface modification may be repeated one or more times. A series of sequential deposition steps may help to provide a DLC coating that has uniform coverage with good adhesion to the substrate surface.

A variety of plasma processing techniques and plasma generating sources are available for DLC surface modification including microwave, electron cyclotron resonance (ECR), microwave coupled with ECR, direct current (DC), RF-glow discharge, inductively coupled plasmas or helicon wave generators, and so forth. US 5,858,477 and US 6,531,182 provide particular examples of systems that are adaptable for depositing DLC on medical devices as described herein.

A typical plasma processing system generally, may include a variable pressure reaction chamber, a power supply, an electrode system, a gas-feeding system, and a vacuum system.

The gas may be passed through a reaction zone and exposed to therein to a radio frequency excitation, microwave excitation, electrodes, etc. The discharge, regardless of which type is employed, i.e. glow, corona, arcing, etc. is maintained at a sufficiently high energy to form desired carbon atom plasma. For polymeric substrates, cold plasma processes are desirable as polymeric substrates can be damaged by high temperatures.

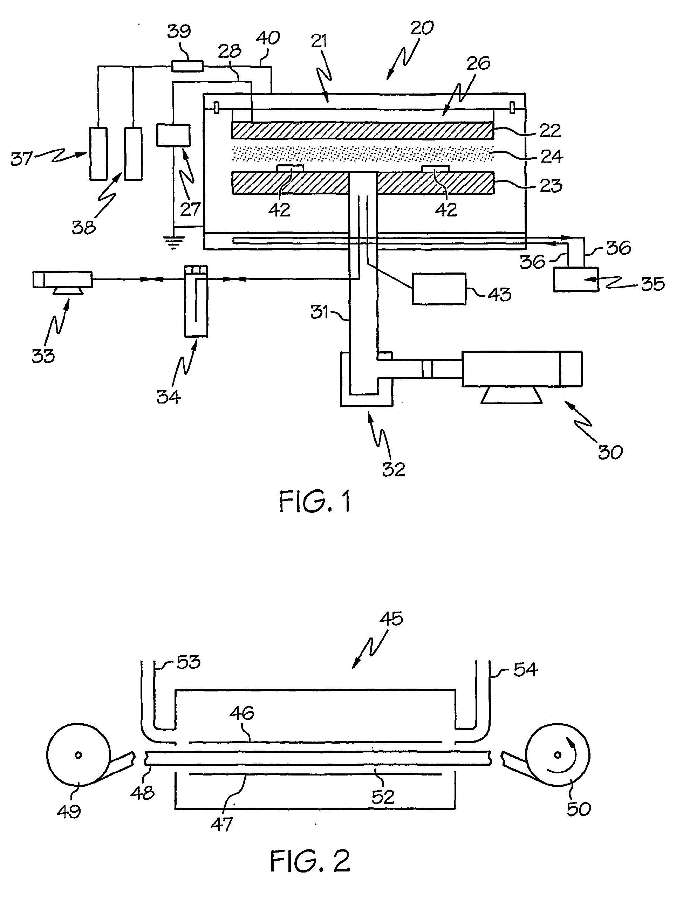

One configuration of a RF-glow discharge system which may be employed herein is shown in Fig 1 of US 6,096,564 and reproduced as Fig. 1 herein. That patent states at col. 5, lines 24-67:
"An example of a cold-plasma reactor system that may be utilized in accordance with the invention is shown schematically at 20 in FIG. 1. The reactor system 20 includes a gas mixing reactor chamber 21 which encloses an upper electrode 22 and a lower electrode 23 between which a plasma reaction region 24 is established. An electrical insulation disk 26 is mounted between the walls of the gas mixing chamber 21 and the upper electrode 22. A radio frequency power supply 27 is connected by lines 28 to the upper electrode 22, while the lower electrode 23 is grounded, as are the walls of the gas mixing chamber 21. A high capacity mechanical vacuum pump 30 is connected by a conduit 31 to the interior of the chamber 21 to selectively evacuate the chamber (a liquid nitrogen trap 32 may be utilized to capture condensates). A further high vacuum mechanical vacuum pump 33 may be connected through another liquid nitrogen trap 34 (for collecting plasma-generated molecular mixtures) to the interior of the chamber 21. An electrical heater 35 is connected to wires 36 to selectively heat the interior of the chamber. A reaction gas reservoir 37 and a monomer reservoir 38 are connected through a control valve 39 by conduits 40 to the interior of the chamber 21 to allow selective introduction of the reaction gas or the monomer into the evacuated chamber.
"In use, a sealable door (not shown in FIG. 1) is opened to allow the substrate materials illustrated at 42 in FIG. 1 to be inserted onto and supported by the lower electrode 23 (and thus also grounded). The door is then closed and sealed, and the high capacity vacuum pump 30 is operated to rapidly withdraw the air within the chamber. The lower capacity, low pressure vacuum pump 33 is then utilized to draw the interior of the chamber down to the desired vacuum base level. When that vacuum level is reached, the valve 39 is operated to selectively allow introduction of the O₂ reaction gas from the reservoir 37 into the reaction chamber at a controlled flow rate to reach a selected gas pressure, and the RF power supply 27 is turned on to provide capacitive coupling between the upper and lower electrodes 22 and 23 and the gas between them to ignite and sustain the plasma in the region 24 between the electrodes. The plasma is maintained at a desired pressure level determined by the introduction of gas from the reactor 37 for a selected period of time, with a pressure gauge 43 being utilized to measure the pressure and control it."

Of course, in accordance with the present invention a DLC modification surface on the substrate is used in place of the O₂ reaction gas mentioned in US 6,096,564.

According to some embodiments of the present invention a substrate to be treated may be located in the region 24 of the device of Fig. 1 and subjected to a surface treatment of a cold plasma of a DLC depositing gas to produce the diamond-like carbon surface. The substrate, or the electrodes, or both may be rotated or otherwise moved in a manner that facilitates uniform exposure of the substrate surface being coated to the DLC depositing plasma.

Figure 2 illustrates another embodiment of a reaction chamber 40 adapted of continuous or semi-continuous deposition on a tubular or wire-like substrate. The reaction chamber 45 employs upper and lower electrodes, 46, 47 respectively, that may be biased by an RF source in a manner similar to that of the electrodes in Fig. 1. A tubular or wire-like substrate 48, such as catheter tubing or guide wire is moved by a suitable motive source from reel 49 to reel 50 during the treatment process, passing through the gap 52 between the electrodes. DLC modification gas is provided to, and removed from, the reactor via ports 53 and 54. In at least some embodiments the chamber is configured so that the flow of gas through the chamber is in the opposite direction of the movement of the substrate 48 through the chamber.

The reaction chamber 45 is suitably operated under vacuum at ambient or near ambient temperature, e.g. 10 - 50°C. The chamber 45 maybe enclosed within a larger reactor housing, not shown, that also encompasses reels 49 and 50 at below ambient pressure.

When DLC modification gas is flowed into the reaction chamber 45 and the RF source is activated, a plasma is generated in the gap causing DLC to be formed on the substrate.

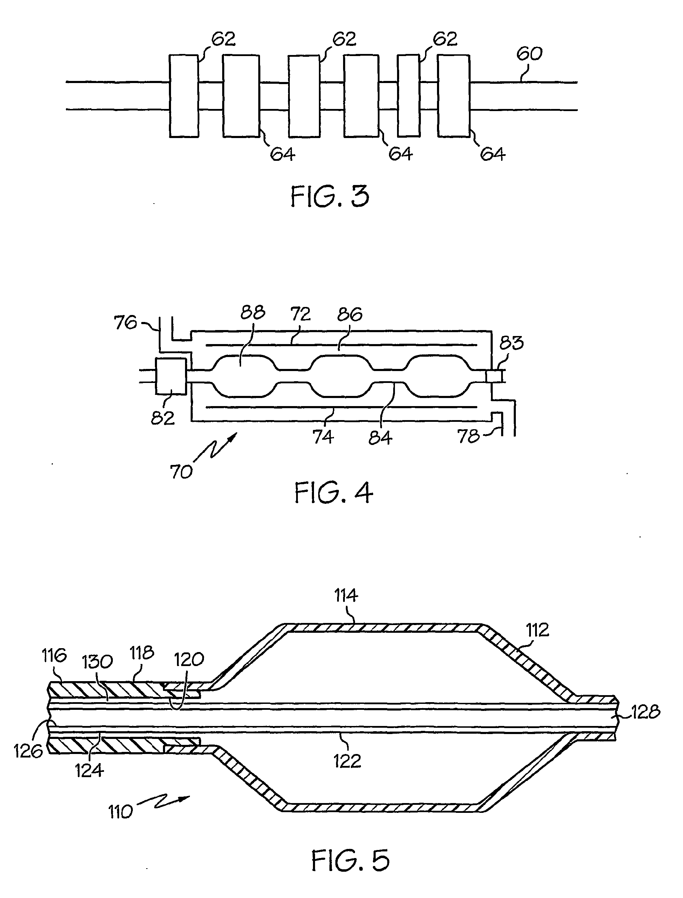

Fig. 3 depicts an alternative arrangement of electrodes that may be employed in a plasma reactor. In this case a tubular or wire-like substrate 60 passes through central openings in a string of alternating cylindrical electrodes 62, 64. Electrodes 62 are biased relative to electrodes 64 by an RF source, not shown.

Fig. 4 depicts a further variation of a reaction chamber. The chamber 70 is provided with upper and lower electrodes 72, 74, respectively, suitably powered and insulated as in Fig. 1. Gas flows in one of the ports 76, 78 and out the other. A motor 82 and bearing structure 83 allows rotation of the substrate 84. In the particular case of Fig. 4 the substrate 84 is a series of balloons that are all blown from a single parison. Such balloons suitably are separated by cutting the parison after the plasma treatment. Alternatively separate balloons may be daisy-chained together to allow for multiple single balloons to be processed concurrently. DLC modification gas may be flowed into the gap 84 between the substrate and the electrodes, or into the internal substrate volume 88, or both. DLC modification gas may be flowed into gap 84 and an inert gas flowed into volume 88, or visa versa. Different plasma gases may be flowed on the outside and inside of the substrate.

In Figure 5 there is shown a distal segment of a balloon catheter 110 that includes a balloon 112 having an outer surface 114. Catheter 110 also includes an outer shaft 116 having outer and inner surfaces 118, 120, respectively, and in inner shaft 122 having outer and inner surfaces 124, 126, respectively. The inner shaft defines a guide wire lumen 128. The space between the inner and outer shafts defines an inflation lumen 130. The balloon 112 is bonded on its proximal end to the outer shaft 116 and on its distal side to the inner shaft 122.

Sliding surfaces of the catheter 110 include at least the inner surface 126 of the inner shaft 122, the outer surface 118 of the outer shaft 116, and the balloon outer surface 114. The inner surface 126 slides over a guide wire during deployment. Outer shaft surface 118 and a portion of the outer balloon surface 114 slide thorough the body vessel, for deployment and removal. In some cases the inner and outer shafts are made movable relative to each other so there may be sliding of inner shaft surface 126 relative to outer shaft surface 120.

To facilitate coating of inner surfaces, the plasma generating gas maybe fed through the substrate as well as around it. In some embodiments of the invention a tubular substrate is provided and the plasma generating gas is fed only through the interior of the device, not around the outside, so the plasma is not generated on the outside of the tube. In other embodiments the interior of the device is sealed, or is separately fed with neutral gas that does not generate plasma, while plasma generating gas is fed around the outside of the substrate. In such cases the coating is provided only on the outside surface of the device. In still another variation, different plasma generating gases may be provided to the interior of the device and the outside. For instance, DLC generating plasma may be provided to the interior of the device and a fluorinating or oxidizing gas may be provided around the outside of the substrate. In such case different coatings may be provided concurrently.

The surface modification with DLC is believed to produce a surface which has low contact adhesion and thus low sliding resistance, relative to the uncoated polymer or metal material upon which it is deposited. The DLC is very thin and thus causes little or no change in the bulk properties of the polymeric material upon which it is deposited. Desirably, the DLC is produced as a layer of 10-10,000 Angstroms.

DLC modification gas may be any gas, or mixture of gases, that deposit DLC from plasma or react with the substrate surface to form a DLC thereon. Hydrocarbon gases are typically used. Acetylene, methane, ethane and butane, cyclohexane, and mixtures thereof are examples. Acetylene is preferred. Other hydrocarbon gases may be useful in some circumstances. In some embodiments the DLC modification gas is a mixture of acetylene and hydrogen. In some embodiments it may be possible to employ a gas such as SF₆, SF₅ or SF₄ to produce a DLC by surface reaction.

DLC is characterized by the existence of sp³ carbon-carbon bonds and the percentage of such bonds on the surface is an indicator of the extent of DLC formed. In some embodiments the sp³ carbon-carbon bonds percentage may be greater than 10%, suitably greater than 15%, for instance from about 30% to about 70%, or even more.

Other gases may also be employed in the plasma processes according to the invention. For example, the presence of a noble gas(es) can facilitate specific reactions by the noble gas metastable energies that are available thus resulting in preferred chemical species and bonding states at the substrate surface. Gases may also be employed simply as diluents, for instance to optimize the DLC deposition from a hydrocarbon gas at the desired chamber pressure. Gases are suitably also employed for chamber purges between treatment cycles or steps. Examples of suitable gases for such uses include, but are not limited to, argon (Ar), hydrogen (H₂), nitrogen (N₂), etc.

Using the process according to the invention, polymer substrate surfaces may be exposed to gaseous plasmas having gases such as nitrogen (N₂), hydrogen (H₂) or argon (Ar) and gases comprising the source of carbon, whereby through this process, carbon atoms become bonded to the polymer surface at the molecular level in the form of DLC.

In some embodiments it may be desirable to treat the substrate surface with an oxygen plasma, e.g. from an Ar/O₂ mixture, or a hydrogen plasma, e.g. from an Ar/H₂ mixture prior to deposition of the DLC coating.

The process according to the invention may be employed to coat both elastomeric and non-elastomeric polymeric materials. Even relatively inert polymeric surfaces such as polyolefinic surfaces, including those formed with polyethylene or polypropylene, may be modified using the method of the present invention.

Examples of polymeric materials suitable for use herein include, but are not limited to, silicone resins, phenolic resins, polyolefins, polyvinyls, polyesters, polyacrylates, polyethers, polyamides including the nylons, polysulfones, cellulosic materials, polystyrene, polyisobutylene, polybutene, polyamide, polycarbonates, polyepoxides, polyacrylonitriles (PAN), block copolymers, etc., copolymers thereof, and mixtures thereof, as well as a wide variety of other polymeric materials not specifically mentioned herein. As used herein, the term "copolymer" shall be used to refer to any polymer formed using two or more monomers including terpolymers and so forth.

Examples of suitable polyolefins include polyethylene, polypropylene as well as copolymers thereof.

Examples of suitable polyester copolymers include, but are not limited to, polyethylene terephthalate, polybutylene terephthalate, and so forth.

Examples of polyamide materials include nylon 6, nylon 6/6, nylon 6/12, nylon 9/12, nylon 6/10, nylon 10, nylon 11, nylon 12, and the like.

Examples of polyether copolymers include polyetheretherketones (PEEK).

Examples of suitable styrenic block copolymers include, but are not limited to, those block copolymers having styrenic endblocks, including, but not limited to, styreneisoprene-styrene (SIS), styrene-butadiene-styrene (SBS), styrene-ethylene/propylene-styrene (SEPS), styrene-isobutylene-styrene (SIBS), styrene-ethylene/butylene-styrene (SEBS), and so forth.

Examples of suitable polyamide block copolymers include, for example, the polyether-block-amides. Examples of polyester block copolymers include, but are not limited to, polyester-block-ester copolymers, polyester-block-ether copolymers and so forth. Polyester and polyamide block copolymer elastomers, and their use as balloon materials are also described in commonly assigned US Patent Nos. 6,406,457, 6,171,278, 6,146,356, 5,951,941, 5,830,182, 5,556,383, 5,112,900.

Examples of suitable polymeric materials particularly suited to forming medical balloons include, but are not limited to, polyesters and copolymers thereof; polyamides and copolymers thereof; polyamide block copolymers, such as those available under the tradename of PEBAX® available from Atofina Chemicals in Philadelphia, PA; polyester block copolymers, polyurethane block copolymers, polyolefins and copolymers thereof, and mixtures thereof. Poly(ester-block-ether) elastomers are available under the tradename of HYTREL® from DuPont de Nemours & Co. and consist of hard segments of polybutylene terephthalate and soft segments based on long chain polyether glycols. These polymers are also available from DSM Engineering Plastics under the tradename of ARNTTEL®. Suitable balloon materials are also described in commonly assigned U.S. Patent Nos. 5549552, 5447497, 5348538, 5550180, 5403340, 6328925, each of which is incorporated by reference herein in its entirety.

Particularly suitable polymeric materials for forming catheter shafts include, but are not limited to, polyolefins such as polyethylene, polyethylene terephthalate, polybutylene terephthalate, poly(ether-block-amide), poly(ester-block-ether), poly(ester-block-ester), and so forth.

Of course, multilayer structures may also be employed herein where two or more polymer layers are formed using different polymeric compositions. The same polymeric composition may also be employed as an alternating layer, for example.

Catheters may be formed of conventional materials of constructions that are described in detail in the art. The proximal shaft section can be manufactured by multi-lumen extrusion using a high-strength polymer such as a polyolefin, polyalkylene terephthalate, nylon, poly(ether-block-amide), polyetheretherketone (PEEK), etc. Coextrusion can be employed to form a multilayer structure as well.

Fibrous material in the form of braiding, weaving, knitting, roving, random, etc. may be provided within a layer, or between layers of the medical devices of the invention.

The above examples and disclosure are intended to be illustrative and not exhaustive. These examples and description will suggest many variations and alternatives to one of ordinary skill in this art. All these alternatives and variations are intended to be included within the scope of the claims, where the term "comprising" means "including, but not limited to." Those familiar with the art may recognize other equivalents to the specific embodiments described herein which equivalents are also intended to be encompassed by the claims. Further, the particular features presented in the dependent claims can be combined with each other in other manners within the scope of the invention such that the invention should be recognized as also specifically directed to other embodiments having any other possible combination of the features of the dependent claims. For instance, for purposes of claim publication, any dependent claim which follows should be taken as alternatively written in a multiple dependent form from all claims which possess all antecedents referenced in such dependent claim if such multiple dependent format is an accepted format within the jurisdiction. In jurisdictions where multiple dependent claim formats are restricted, the following dependent claims should each be also taken as alternatively written in each singly dependent claim format which creates a dependency from an antecedent-possessing claim other than the specific claim listed in such dependent claim.

## Claims

1. A medical device comprising a catheter having a tubular polymeric portion comprising a guide wire lumen, having an inner lumen wall, the tubular portion being only of polymeric material, **characterized in that** said inner lumen wall is coated with a diamond hard coating (DLC), wherein the tubular polymeric portion further has an exterior surface and the exterior surface has a coating thereon obtained by exposing the surface to a plasma, and wherein said plasma comprised a fluorinating or oxidizing gas.

2. A method of treating a surface of a polymeric medical device comprising a catheter substrate comprising:
a) enclosing the polymeric medical device substrate in a reaction chamber with the surface to be coated exposed to the environment of the reaction chamber;
b) evacuating the reaction chamber to a base level;
c) supplying a hydrocarbon DLC depositing gas to and establishing a selected hydrocarbon DLC depositing hydrocarbon gas pressure in the reaction chamber; and
d) igniting a cold plasma in the gas in the chamber and exposing the substrate surface to the plasma for a selected period of time sufficient to form a layer of DLC thereon,
**characterized in that** the medical device substrate is a tubular substrate formed only of polymeric material and having an inner lumen, the hydrocarbon DLC depositing gas is fed at least through said lumen, the plasma is ignited in the gas within said lumen, and the medical device substrate has an outer surface and a different film forming plasma generating gas comprising oxygen and fluorine is fed around the outer surface.

3. A method as in claim 2 wherein the medical device substrate comprises a catheter tube or a balloon.

## Patentansprüche

1. Medizinische Vorrichtung umfassend einen Katheter mit einem röhrenförmigen polymeren Abschnitt, der ein Lumen für einen Führungsdraht mit einer inneren Lumenwand umfasst, wobei der röhrenförmige Abschnitt nur aus polymerem Material besteht, **dadurch gekennzeichnet, dass** die innere Lumenwand mit einer Hartbeschichtung aus diamantartigem Kohlenstoff (DLC) beschichtet ist, wobei der röhrenförmige polymere Abschnitt ferner eine äußere Oberfläche aufweist und die äußere Oberfläche eine Beschichtung darauf hat, die erhalten wurde, indem die Oberfläche einem Plasma ausgesetzt wurde, wobei das Plasma ein fluorierendes oder oxidierendes Gas enthielt.

2. Verfahren zum Behandeln einer Oberfläche einer polymeren medizinischen Vorrichtung umfassend ein Kathetersubstrat, umfassend:
a) Einschließen des Substrats der polymeren medizinischen Vorrichtung in einer Reaktionskammer, wobei die zu beschichtende Oberfläche der Umgebung der Reaktionskammer ausgesetzt ist;
b) Evakuieren der Reaktionskammer auf ein Basisniveau;
c) Einleiten eines DLC abscheidenden Kohlenwasserstoffgases in die Reaktionskammer und Herstellen eines ausgewählten Drucks des DLC abscheidenden Kohlenwasserstoffgases darin; und
d) Zünden eines kalten Plasmas in dem Gas in der Kammer und Aussetzen der Substratoberfläche dem Plasma einen ausgewählten Zeitraum lang, der ausreicht, darauf eine Schicht von DLC zu bilden,
**dadurch gekennzeichnet, dass** das Substrat der medizinischen Vorrichtung ein röhrenförmiges, nur aus polymerem Material erzeugtes Substrat mit einem inneren Lumen ist, das DLC abscheidende Kohlenwasserstoffgas mindestens durch das Lumen eingeleitet wird, das Plasma in dem Gas innerhalb des Lumens gezündet wird und das Substrat der medizinischen Vorrichtung eine äußere Oberfläche aufweist und ein anderes, filmbildendes Plasma erzeugendes Gas umfassend Sauerstoff und Fluor um die äußere Oberfläche herum eingespeist wird.

3. Verfahren gemäß Anspruch 2, wobei das Substrat der medizinischen Vorrichtung eine Katheterröhre oder einen Ballon umfasst.

## Revendications

1. Dispositif médical comprenant un cathéter avec une portion tubulaire polymère qui comprend une lumière pour un fil guide et qui a une paroi de lumière intérieure, la portion tubulaire étant faite seulement de matériau polymère, **caractérisé en ce que** la paroi de lumière intérieure est revêtue par un revêtement dur à base de carbone sous forme de diamant amorphe (DLC), la portion tubulaire polymère ayant en outre une surface extérieure et la surface extérieure ayant sur elle-même un revêtement qui était obtenu par l'exposition de la surface à un plasma et que ledit plasma comprenait un gaz fluorissant ou oxydant.

2. Procédé pour traiter une surface d'un dispositif médical polymère comprenant un substrat de cathéter, le procédé comprenant:
a) enfermer le substrat du dispositif médical polymère dans une chambre de réaction, la surface qu'on veut traiter étant exposée à l'environnement de la chambre de réaction;
b) évacuer la chambre de réaction à un niveau de base;
c) amener un gaz hydrocarbure déposant DLC à la chambre de réaction et établir une pression sélectionnée du gaz hydrocarbure déposant DLC dans la chambre de réaction; et
d) allumer un plasma froid dans la chambre et exposer la surface du substrat au plasma pour un période de temps sélectionné qui suffit pour former une couche de DLC sur cela,
**caractérisé en ce que** le substrat du dispositif médical est un substrat tubulaire formé seulement de matériau polymère et a une lumière intérieure, le gaz hydrocarbure déposant DLC est fourni au moins à travers cette lumière, le plasma est allumé dans le gaz à l'intérieur de cette lumière et le substrat du dispositif médical a une surface extérieure, et un autre gaz générant du plasma et formant un film et comprenant de l'oxygène et de la fluorure est fourni autour de la surface extérieure.

3. Procédé selon la revendication 2, dans lequel le substrat du dispositif médical comprend un tube de cathéter ou un ballonnet.
